# EUROPÄISCHE PATENTSCHRIFT

(11) **EP 2 812 651 B1**
(45) Veröffentlichungstag und Bekanntmachung des Hinweises auf die Patenterteilung: **30.09.2020**
(21) Anmeldenummer: 13708645.0
(22) Anmeldetag: 04.02.2013
(51) Int. Cl.: G01B 21/04, A61C 9/00, A61B 5/107, G01B 11/25

(54) **VERFAHREN ZUM DARSTELLEN OPTISCH ERMITTELTER OBERFLÄCHENGEOMETRIEN**
METHOD FOR DISPLAYING OPTICALLY DETERMINED SURFACE GEOMETRIES
PROCÉDÉ POUR REPRÉSENTER DES GÉOMÉTRIES DE SURFACES OBTENUES PAR VOIE OPTIQUE

(30) Priorität: 06.02.2012 DE 102012100959
(43) Veröffentlichungstag der Anmeldung: 17.12.2014
(73) Patentinhaber: a.tron3d GmbH, 9020 Klagenfurt am Wörthersee (AT)
(72) Erfinder: JESENKO, Jürgen, 9584 Finkenstein (AT)
(74) Vertreter: Beer & Partner Patentanwälte KG
(86) Internationale Anmeldenummer: PCT/AT2013/000016
(87) Internationale Veröffentlichungsnummer: WO 2013/116879

(56) Entgegenhaltungen:
- US-A1- 2007 172 112

## Beschreibung

Die Erfindung betrifft ein Verfahren zum Darstellen wenigstens einer Eigenschaft einer optisch ermittelten Oberflächengeometrie wenigstens eines dreidimensionalen Objektes, insbesondere eines Zahnes, auf einem Display, insbesondere einem Computerbildschirm.

Derartige Verfahren sind beispielsweise aus der AT 508 563 B oder der US 2007/172112 A1 bekannt. Der Anwendungsbereich der Erfindung erstreckt sich dabei auf die digitale Aufnahme von Zahn- und Kieferabdrücken, die Hilfestellung bei der Diagnose, die Überwachung von Zahnbehandlungen sowie die zuverlässige Kontrolle von eingesetzten Implantaten. Neben weiteren Einsatzgebieten im Bereich der Medizin- und Industrietechnik, beispielsweise im Bereich der Endoskopie, können auch Objekte stereometrisch vermessen und dargestellt werden, die schwer zugänglich sind.

Nachteilig ist dabei, dass zwar eine dreidimensionale Geometrie dargestellt wird, dem Nutzer aber keine weiteren Informationen zur Verfügung gestellt werden, die ihm bei der Handhabung des Gerätes als Unterstützung zur Gewinnung von möglichst genauen Oberflächengeometrien dienen.

Ziel der Erfindung ist es daher, die Darstellung von optisch ermittelten Oberflächengeometrien derart zu verbessern, dass dem Nutzer zusätzliche Informationen zur Verfügung gestellt werden, die ihm die Handhabung derart erleichtern, dass er gezielt die erfasste Oberflächengeometrie, je nach Anforderung, vollständig oder auch nur bereichsweise verbessern kann.

Diese Aufgabe wird mit einem Verfahren gemäß Anspruch 1 gelöst.

Eine Menge wird dabei im mathematischen Sinn verstanden, kann also beispielsweise auch nur ein Element enthalten. Eine leere Menge wäre in diesem Sinne zwar auch denkbar, würde aber keinen technischen Sinn erfüllen, da der Menge ohne Elemente keine Eigenschaft mehr zugewiesen werden kann.

Gemäß einer bevorzugten Ausführungsform der Erfindung wird die Oberflächengeometrie mittels Stereometrie erfasst. Diese ist für das Verfahren besonders gut geeignet, da hier aufgrund der Transformationsmatrix, durch welche aufgenommene, zweidimensionale Daten in eine dreidimensionale Oberflächengeometrie umgewandelt werden, mehr Daten eines Bereichs der Oberflächengeometrie auch immer zu mehr Genauigkeit führen.

In einer weiteren, bevorzugten Ausführungsform der Erfindung erfolgt die grafische Darstellung unterschiedlicher Eigenschaften durch unterschiedliche Farben. Dies ist besonders vorteilhaft, da Farben im allgemeinen bereits ohne einen Schlüssel bzw. eine Anleitung zur Interpretation intuitiv erfasst werden können. Ein einfaches Beispiel ist die Assoziation von Grün mit "gut" und Rot mit "schlecht". So können beispielsweise Mengen von Bereichen mit einer großen Anzahl von Daten, also einer hohen Genauigkeit, in grüner Farbe und Mengen von Bereichen mit einer geringen Anzahl von Daten in roter Farbe dargestellt werden. Wenn beispielsweise Bereiche rot eingefärbt sind, so erhält der Nutzer daraus die Information, dass hier weitere Daten benötigt werden, er den Bereich also beispielsweise ein weiteres Mal aufnehmen muss, um zur gewünschten Aufnahmegenauigkeit zu gelangen. Zusätzlich oder alternativ kann auch eine Farbe bestimmt werden, mit welcher dem Nutzer signalisiert wird, dass in dem betreffenden Bereich bereits eine optimale Genauigkeit erreicht ist, weitere Aufnahmen also keine nennenswerte Verbesserung der Genauigkeit der dargestellten Oberflächengeometrie mehr bewirken. Selbstverständlich sind auch andere Arten der Darstellung denkbar. So können beispielsweise Bereiche, die eine gewünschte Genauigkeit erreicht haben, durch Häkchen repräsentiert oder gekennzeichnet werden.

Erfindungsgemäß sind die Bereiche einzelne Voxel, also Volumen-Pixel, der Oberflächengeometrie. In diesem Fall könnte also beispielsweise bei Mengen mit nur einem Element das Voxel selbst eingefärbt werden um die Eigenschaft wiederzugeben. Für Mengen mit mehr als einem Element wäre beispielsweise eine Menge denkbar, die alle Voxel eines Zahnes umfasst. In diesem Fall wäre eine zusätzliche Darstellung außerhalb der Darstellung der eigentlichen Oberflächengeometrie denkbar. So könnten beispielsweise repräsentativ Flächen oder Objekte an einem Rand der Darstellung eingefärbt oder andersartig graphisch verändert werden, wenn die Anzahl an Daten als Kriterium für alle Voxel eines Zahnes erfüllt ist.

Gemäß einer ganz besonders bevorzugten Ausführungsform der Erfindung ist die Anzahl die Summe der Aktualisierungen des definierten Bereichs. Unter Aktualisierung ist dabei eine verbesserte Definition des Bereichs z.B. durch wiederholte Scans mittels wenigstens einer Kamera zu verstehen. Da sich bei stereometrischen Verfahren durch mehr Aktualisierungen mehr Daten ergeben und diese zu mehr Genauigkeit führen, ist eine unmittelbare Zuordnung der Anzahl der Aktualisierungen zur graphischen Darstellung ein besonders einfacher und damit für eine Recheneinheit besonders Ressourcen schonender Weg um eine Eigenschaft einer optisch ermittelten Oberflächengeometrie darzustellen.

In einem einfachen Beispiel, bei dem beispielsweise lediglich die Anzahl der Aktualisierungen, welche von einem definierten Bereich gemacht wurden, das Kriterium ist, können beispielsweise 7 oder mehr Aktualisierungen als optimal angesehen werden. Mengen mit Bereichen, von denen also bereits wenigstens 7 Aktualisierungen gemacht wurden, kann die Eigenschaft "optimal" zugewiesen werden und diese Eigenschaft kann beispielsweise in weißer Farbe dargestellt werden. Um die gewünschte Qualität der Aufnahme, beispielsweise die Eigenschaft "gut", zu erreichen, können beispielsweise bereits 5 bis 6 Aktualisierungen genügen. Mengen mit Bereichen, von denen also 5 bis 6 Aktualisierungen gemacht wurden, könnte man beispielsweise Grün darstellen. Mengen von Bereichen von denen für die gewünschte Qualität nur eine unzureichende Anzahl von Aktualisierungen gemacht wurde, denen man also beispielsweise die Eigenschaften "ungenügend" oder "mangelhaft" zuweisen könnte, könnten zum Beispiel in Rot oder Blau dargestellt werden. Blau stünde dabei für die etwas bessere Qualität von 3 bis 4 Aktualisierung, also die Eigenschaft "mangelhaft". Rot ließen sich dementsprechend Mengen von Bereichen darstellen, von denen nur 1 bis 2 Aktualisierungen gemacht wurden, denen also die Eigenschaft "ungenügend" zugewiesen wurde. Mengen von Bereichen von denen noch gar keine Aktualisierung gemacht wurden, die aber (beispielsweise durch Extrapolation) dennoch dargestellt werden, ließe sich beispielsweise die Eigenschaft "leer" zuweisen, die man beispielsweise Schwarz darstellen könnte.

Erfindungsgemäß wird zunächst ermittelt, wie viele aufgenommene Daten insgesamt für einen definierten Bereich der ermittelten Oberflächengeometrie zur Verfügung stehen. Die Definition der Bereiche erfolgt dabei beispielsweise über die Unterteilung der Aufnahme in sogenannte Voxel bzw. Gruppen von Voxeln, beispielsweise 4³ Voxel. Alternative Bereichsdefinitionen, die sich beispielsweise aus Mustern ergeben, die zur Unterstützung der optischen Ermittlung der Oberflächengeometrie eines Objektes auf das Objekt projiziert oder anderweitig aufgebracht wurden, sind ebenso denkbar. Erfindungsgemäß ist der definierte Bereich ein Voxel.

Ausführungsformen die zusätzlich oder alternativ eine andere grafische Darstellung als unterschiedliche Farben vorsieht, also beispielsweise unterschiedliche Intensitäten ein und der selben Farbe oder unterschiedliche Körnungen, sind dabei ebenso im Sinne der Erfindung.

Weitere bevorzugte Aus- und Durchführungsformen der Erfindung sind Gegenstand der übrigen abhängigen Ansprüche.

Die Erfindung wird in der Folge unter Bezugnahme auf die Zeichnungen weiter erläutert. Es zeigt:
- Fig. 1: eine nicht unter den Schutzbereich fallende Darstellung einer Gruppe von Zähnen mit zwei repräsentativen Darstellungen der Zähne, welche einzeln oder gemeinsam dargestellt werden können,
- Fig. 2: eine weitere nicht unter den Schutzbereich fallende Darstellung der Gruppe von Zähnen teilweise eingefärbt,
- Fig. 3: eine weitere nicht unter den Schutzbereich fallende Darstellung der Gruppe von Zähnen mit einer alternativen Art der Einfärbung und
- Fig. 4: die Gruppe von Zähnen mit einer dritten, erfindungsgemäßen Art der Einfärbung.

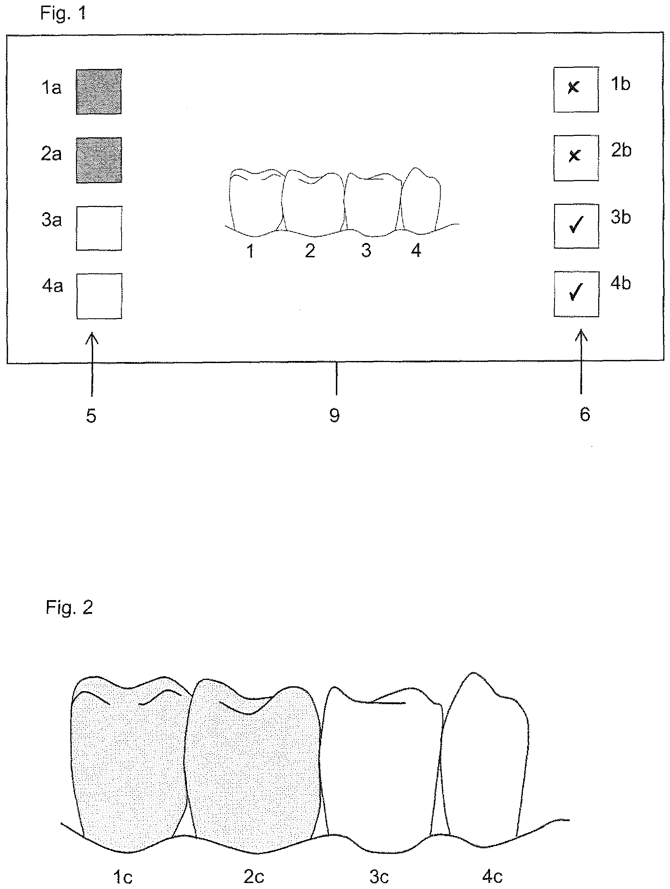

Die Fig. 1 zeigt eine nicht unter den beanspruchten Schutzbereich fallende Ausführungsform, bei welcher dreidimensionale Objekte 1, 2, 3, 4, deren Oberflächengeometrie ermittelt wird, Zähne sind, mit zwei möglichen repräsentativen Darstellungen 5, 6, wobei die Menge der definierten Bereiche die Menge aller Voxel jeweils eines Zahnes ist. Dies wird auf einem Display 9 abgebildet. Hierbei wird als Kriterium die kleinste Anzahl an optisch ermittelten Daten, welche für eine gewünschte Genauigkeit notwendig sind, definiert. So können bei Erreichen der gewünschten Genauigkeit in einer ersten repräsentativen Darstellung 5 beispielsweise Vierecke 1a, 2a, 3a, 4a, welche die Zähne 1, 2, 3, 4 repräsentieren, ihre Farbe wechseln. Im Dargestellten Beispiels haben der erste und zweite Zahn 1, 2 noch nicht die gewünschte Genauigkeit erreicht. Die repräsentativen ersten beiden Vierecke 1a, 2a werden in der Zeichnung in Grau, auf dem tatsächlichen Bildschirm aber beispielsweise in Rot, dargestellt. Für den dritten und vierten Zahn 3, 4 ist das Kriterium bereits erfüllt, die repräsentativen Vierecke zu diesen Zähnen 3a, 4a sind in der Zeichnung in Weiß, auf dem tatsächlichen Bildschirm aber beispielsweise in Grün, dargestellt. Analog sind in einer alternativen zweiten repräsentativen Darstellung Vierecke 1b, 2b, 3b, 4b mit Häkchen oder X versehen. Verschiedenste Ausführungsformen in denen statt Farben beispielsweise Zahlen die Genauigkeit der Aufnahme repräsentieren, also beispielsweise eine Skala von 1 bis 10, oder in der statt Vierecken 1a, 2a, 3a, 4a, 1b, 2b, 3b, 4b anschaulichere Repräsentationen der Objekte, beispielsweise zahnförmige Flächen, gewählt werden sind ebenso im Sinne der Erfindung. Alternativ oder zusätzlich können wie in Fig. 2 zu sehen ist, auch die dargestellten Objekte 1c, 2c, 3c, 4c selbst eingefärbt werden.

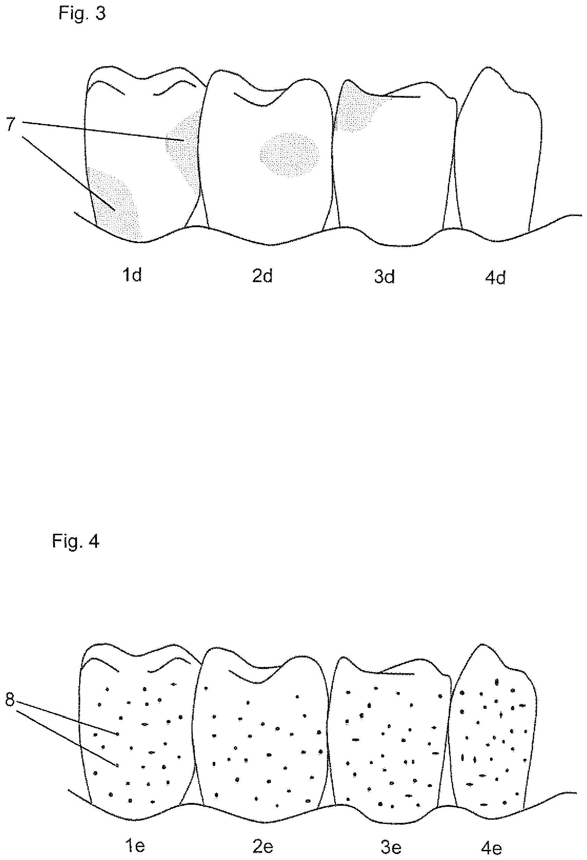

Die Fig. 3 zeigt eine nicht unter den beanspruchten Schutzbereich fallende Ausführungsform, bei der, ähnlich wie in der Fig. 2 die dargestellte Geometrie selbst eingefärbt wird. Allerdings enthalten die Mengen weniger Elemente. So können wie in Fig. 3 gezeigt zum Beispiel Abschnitte 7 der Objekte 1d, 2d, 3d, 4d eingefärbt werden, was die Handlungsanweisung für den Nutzer präziser macht. Statt einer Anweisung, welche Zähne noch besser erfasst werden sollen, erhält er nun die genauere Anweisung, beispielsweise welche Bereiche oder aus welcher Richtung er die Objekte noch weiter erfassen muss, um zur gewünschten Genauigkeit zu gelangen.

Die Fig. 4 zeigt eine erfindungsgemäße Ausführungsform, in der die Mengen auf einzelne Voxel 8 eingeschränkt wurden. Der Nutzer erhält also ähnliche Anweisungen wie in der Figur 3, nur mit noch mehr Details. Bei Ausführungsformen, in denen mehr Kriterien als die beiden beispielhaft gezeigten definiert werden, wird die Handlungsanweisung entsprechend umfassender.

## Patentansprüche

1. Verfahren zum Darstellen wenigstens einer Eigenschaft einer optisch ermittelten Oberflächengeometrie wenigstens eines dreidimensionalen Objektes, insbesondere eines Zahnes, auf einem Display, insbesondere einem Computerbildschirm, **dadurch gekennzeichnet, dass** bei einem definierten Bereich der Oberflächengeometrie geprüft wird, ob ein definiertes Kriterium erfüllt ist, dass der definierte Bereich ein Voxel ist, dass eine Anzahl von optisch ermittelten Daten des definierten Bereiches ein Kriterium ist, dass einer Menge von definierten Bereichen der Oberflächengeometrie eine Eigenschaft zugewiesen wird, wenn alle Bereiche der Menge das Kriterium erfüllen, dass die Eigenschaft in einer definierten Weise grafisch dargestellt wird und dass unterschiedliche Eigenschaften unterschiedlich grafisch dargestellt werden.

2. Verfahren nach Anspruch 1, **dadurch gekennzeichnet, dass** die Oberflächengeometrie mittels Stereometrie erfasst wird.

3. Verfahren nach Anspruch 1 oder 2, **dadurch gekennzeichnet, dass** die grafische Darstellung unterschiedlicher Eigenschaften durch unterschiedliche Farben erfolgt.

4. Verfahren nach einem der Ansprüche 1 bis 3, **dadurch gekennzeichnet, dass** die Anzahl eine Summe von Aktualisierungen des definierten Bereichs ist.

## Claims

1. Method for displaying at least one property of an optically determined surface geometry of at least one three-dimensional object, in particular a tooth, on a display, in particular a computer screen, **characterized in that** in a defined region of the surface geometry, it is checked whether a defined criterion is met, **in that** the defined region is a voxel, **in that** a number of optically determined data of the defined region is a criterion, **in that** a property is assigned to a set of defined regions of the surface geometry if all regions of the set meet the criterion, **in that** the property is displayed graphically in a defined manner, and **in that** different properties are displayed graphically in different ways.

2. Method according to claim 1, **characterized in that** the surface geometry is detected by means of stereometry.

3. Method according to either claim 1 or claim 2, **characterized in that** different properties are displayed graphically by means of different colors.

4. Method according to any of claims 1 to 3, **characterized in that** the number is a sum of updates of the defined region.

## Revendications

1. Procédé de représentation d'au moins une propriété d'une géométrie de surface déterminée par voie optique d'au moins un objet tridimensionnel, en particulier d'une dent, sur un affichage, en particulier un écran d'ordinateur, **caractérisé en ce que** l'on vérifie, dans une zone définie de la géométrie de surface, si un critère défini est satisfait, **en ce que** la zone définie est un voxel, **en ce qu'**un nombre déterminé de données par voie optique de la zone définie est un critère, **en ce qu'**une propriété est attribuée à un ensemble de zones définies de la géométrie de surface si toutes les zones de l'ensemble satisfont le critère, **en ce que** la propriété est représentée graphiquement d'une manière définie et **en ce que** différentes propriétés sont représentées graphiquement différemment.

2. Procédé selon la revendication 1, **caractérisé en ce que** la géométrie de surface est enregistrée par stéréométrie.

3. Procédé selon la revendication 1 ou 2, **caractérisé en ce que** la représentation graphique de différentes propriétés est effectuée au moyen de différentes couleurs.

4. Procédé selon l'une des revendications 1 à 3, **caractérisé en ce que** le nombre est une somme de mises à jour de la zone définie.
